# FASCICULE DE BREVET EUROPEEN

(11) **EP 3 481 892 B1**
(45) Date de publication et mention de la délivrance du brevet: **07.06.2023**
(21) Numéro de dépôt: 17739523.3
(22) Date de dépôt: 04.07.2017
(51) Int. Cl.: C08J 11/16, C08J 11/24, C07C 67/48, C07C 69/82

(54) **PROCÉDÉ DE DÉPOLYMÉRISATION D'UN POLYESTER COMPRENANT DU POLYÉTHYLÈNE TÉRÉPHTALATE OPAQUE**
VERFAHREN ZUR ENTPOLYMERISIERUNG EINES POLYESTERS MIT OPAKEM POLYETHYLENTEREPHTHALAT
METHOD FOR THE DEPOLYMERISATION OF A POLYESTER COMPRISING OPAQUE POLYETHYLENE TEREPHTHALATE

(30) Priorité: 05.07.2016 FR 1656423
(43) Date de publication de la demande: 15.05.2019
(73) Titulaire: IFP Energies nouvelles, 92852 Rueil-Malmaison Cedex (FR)
(72) Inventeur: CHARRA, Cyprien, 69003 LYON (FR); FAVRE, Frederic, 69005 LYON (FR); MEKKI-BERRADA, Adrien, 42000 ST ETIENNE (FR); THINON, Olivier, 42300 ROANNE (FR)
(74) Mandataire: IFP Energies nouvelles
(86) Numéro de dépôt international: PCT/EP2017/066577
(87) Numéro de publication internationale: WO 2018/007356

(56) Documents cités:
- EP-A1- 1 130 011
- WO-A1-2016/096767
- US-A- 3 907 868
- US-A1- 2004 147 624

## Description

### DOMAINE TECHNIQUE DE L'INVENTION

L'invention concerne un procédé de dépolymérisation d'un polyester, en particulier le polyester téréphtalate, en vue de son recyclage dans une unité de polymérisation.

### ART ANTÉRIEUR

Le recyclage chimique du polyéthylène téréphtalate (PET) a fait l'objet de nombreux travaux visant à décomposer le PET récupéré sous forme de déchets en monomères qui pourront de nouveau être utilisés comme charge d'un procédé de polymérisation.

De nombreux polyesters sont issus de circuits de collecte et de tri de matières. En particulier, le PET peut provenir de la collecte de bouteilles, barquettes, films, résines et/ou fibres constitués de PET. Le polyester issu de filières de collecte et de recyclage est appelé polyester recyclé.

Le PET recyclé peut être classé en quatre grandes catégories :
- le PET clair, constitué majoritairement de PET incolore transparent (en général au moins 60% poids) et de PET coloré azuré, qui ne contient pas de pigments et peut être envoyé dans des procédés de recyclage mécanique,
- le PET foncé, ou coloré (vert, rouge,..), qui peut contenir généralement jusqu'à 0,1% poids de colorants ou pigments mais reste transparent, ou translucide,
- le PET opaque, qui contient une quantité significative de pigments à des teneurs variant typiquement entre 0,25 et 5% pds, utilisés pour opacifier le polymère, utilisé de manière grandissante par exemple pour la fabrication de contenants alimentaires, comme les bouteilles de lait, dans la composition de flacons cosmétiques, phytosanitaires ou de colorants.
- le PET multicouches, qui comporte des couches de plastiques autres que le PET ou une couche de PET recyclé entre des couches de PET vierge, c'est à dire n'ayant pas subi de recyclage, ou un film d'aluminium par exemple. Ce PET est utilisé après thermoformage pour faire des emballages tels que des barquettes.

Les filières de collecte permettant d'alimenter les filières de recyclage sont structurées différemment en fonction des pays. Elles évoluent de manière à maximiser la quantité de plastique valorisé dans les déchets en fonction de la nature et de la quantité des flux et des technologies de tri.

La filière de recyclage de ces différents flux est en général constituée d'une première étape de conditionnement sous forme de paillette dans laquelle des balles d'emballage brut sont lavées, purifiées et triées, broyées puis de nouveau purifiées et triées pour produire un flux de paillettes contenant en général moins de 2% d'impuretés (verre, métaux, autres plastiques, bois, papier carton, éléments minéraux), préférentiellement moins de 1% d'impuretés.

Les paillettes de PET clair peuvent ensuite subir une étape d'extrusion-filtration permettant de produire des extrudés qui sont ensuite réutilisables en mélange avec du PET vierge pour faire de nouveaux produits (bouteilles, fibres, films). Une étape de polymérisation sous vide à l'état solide (connu sous l'acronyme SSP) est nécessaire pour les usages alimentaires. Ce type de recyclage est appelé recyclage mécanique.

Les paillettes de PET foncé ou coloré sont recyclables mécaniquement également. Cependant, la coloration des extrudés formés à partir des flux colorés limite les usages et ce PET est le plus souvent utilisé pour produire des fibres ou des lanières d'emballage. Les débouchés sont donc plus limités.

La présence de PET opaque contenant des teneurs en pigments importantes pose des problèmes aux recycleurs car le PET opaque altère les propriétés mécaniques du PET recyclé. Le PET opaque est actuellement collecté avec le PET coloré et se retrouve dans le flux de PET coloré. Compte tenu du développement des usages du PET opaque, les teneurs en PET opaque dans le flux de PET coloré sont actuellement comprises entre 5-10% et augmentent. D'ici quelques années, il sera possible d'atteindre des teneurs en PET opaque dans le flux de PET coloré supérieures à 20%. Or, il a été montré qu'au-delà de 10-15% de PET opaque dans les flux de PET coloré, les propriétés mécaniques du PET recyclé sont altérées et empêchent le recyclage sous forme de fibres, principal débouché de la filière pour le PET coloré.

Les principaux pigments utilisés sont des oxydes métalliques comme TiO₂, CoAl₂O₄, Fe₂O₃, des silicates, des polysulfides, et du noir de carbone. Les pigments sont des particules de taille comprise généralement entre 0,1 et 10 µm, et en majorité entre 0,4 et 0,8 µm. L'élimination totale de ces pigments par filtration, nécessaire pour envisager un recyclage du PET opaque, est techniquement difficile. En effet, d'une part ces particules sont extrêmement colmatantes, d'autre part certains pigments sont connus pour catalyser la réaction de polymérisation du PET dans les conditions opératoires des opérations de séparation, ce qui accroît les risques de colmatage des filtres avec les polymères produits au sein desdits filtres.

Les colorants utilisés sont de différentes natures et contiennent souvent des hétéroatomes de type O et N, et des insaturations conjuguées, comme par exemple des fonctions quinone, methine, azo, ou des molécules comme la pyrazolone et la quinophtalone.

Le recyclage des PET colorés ou opaques est donc extrêmement délicat.

Le brevet EP0865464 décrit un procédé de recyclage par dépolymérisation de polyesters comprenant les étapes de dépolymérisation en présence d'un diol, d'évaporation du diol, de dissolution du mélange dans un solvant chaud, de filtration et de précipitation de la solution filtrée, le précipité pouvant ensuite servir pour la préparation d'un nouveau polymère. Ce document décrit que les monomères et oligomères peuvent être séparés dans un évaporateur à film raclé (thin film evaporator selon le terme anglais), sans toutefois préciser dans quelles conditions doit être opéré cet évaporateur. Ce brevet n'aborde pas non plus les problématiques liées à la nature du PET traité.

Le brevet JP3715812 décrit l'obtention de BHET raffiné à partir de PET. La dépolymérisation est suivie d'une étape de pré-purification par refroidissement, filtration, adsorption et traitement sur résine échangeuse d'ions, présentée comme très importante, réalisée avant l'évaporation du glycol et la purification du BHET. La pré-purification permet d'éviter la re-polymérisation du BHET dans les étapes subséquentes de purification. Ce procédé fonctionne parfaitement tant que la charge contient des impuretés solides simples à séparer (plastiques autres que PET, résidus solides). En revanche, le passage par une étape de filtration et résine échangeuse d'ions est extrêmement problématique lorsque la charge comprend une quantité importante de très petites particules solides, à l'instar des pigments, ce qui est le cas lorsque la charge traitée comprend du PET opaque, en particulier en proportions conséquentes (plus de 10% poids de PET opaque).

Le brevet EP0865464 décrit le recyclage par dépolymérisation de polyesters par un diol, suivie par une étape d'évaporation du diol, puis par la dilution dans un solvant à chaud. Cette dilution à chaud permet de séparer les impuretés de taille supérieure à 50µm par filtration. La solution traitée est ensuite refroidie et les constituants précipités repolymérisés. L'étape de filtration permet de retirer les impuretés insolubles. La faible proportion de pigments dans du PET coloré permet une séparation par filtration. Toutefois, cette technologie ne pourrait fonctionner avec la quantité de pigments présents dans du PET opaque, ces pigments colmatant rapidement le filtre.

Le brevet FR2103115 traite de la purification du BHET par distillation avec un temps de séjour très court afin d'éviter la repolymérisation du BHET, principalement en vue d'éliminer les impuretés issues de la réaction de l'acide téréphtalique et de l'oxyde d'éthylène. Ce document enseigne qu'il est pertinent d'opérer la séparation du BHET à température relativement élevée (200-350°C) afin de minimiser le temps de séjour dans la distillation. Ce document ne traite pas de la présence des impuretés solides autres, telles que les pigments. Or à température élevée, ces pigments vont largement favoriser la polymérisation du BHET.

US2004147624 A décrit un procédé de recyclage de bouteilles PET usées, qui comprend également des bouteilles PET colorées.

EP1130011 A1 décrit un procédé pour éliminer l'oxyde de titane d'un produit de décomposition du polyester obtenu par une étape de solvolyse de l'éthylène glycol.

### OBJET ET INTÉRÊT DE L'INVENTION

L'invention a pour objet un procédé de dépolymérisation d'une charge polyester comprenant du PET opaque, ledit procédé comprenant au moins les étapes suivantes :
a) une étape de conditionnement alimentée par ladite charge polyester ;
b) une étape de dépolymérisation par glycolyse alimentée au moins par l'effluent de l'étape
   a) et par un appoint de diol, opérée à une température comprise entre 200 et 400°C, avec de 1 à 20 moles de diol par mole de diester dans ladite charge polyester et un temps de séjour du polyester compris entre 0,1 et 5 h ;
c) une étape de séparation du diol alimentée au moins par l'effluent de l'étape b), opérée à une température comprise entre 100 et 250°C, à une pression inférieure à celle de l'étape b) et produisant un effluent diol et un effluent riche en monomères liquide ;
d) une étape de séparation de l'effluent riche en monomères liquides issu de l'étape c) en un effluent impuretés lourdes et un effluent monomères pré-purifié opérée à une température inférieure à 250°C et une pression inférieure à 0,001 MPa avec un temps de séjour liquide inférieur à 10 min et
e) une étape de décoloration de l'effluent monomères pré-purifié, opérée à une température comprise entre 100 et 250°C, et à une pression comprise entre 0,1 et 1,0 MPa en présence d'un adsorbant et produisant un effluent monomères purifié.

Un intérêt de l'invention est de pouvoir traiter des polyesters comprenant des pigments et des colorants, en particulier des PET azurés, colorés, opaques, voire multi-couche.

Le procédé selon l'invention, apte à traiter du PET opaque, permet de retirer les pigments et colorants et de revenir au monomère par réaction chimique. Ce monomère est ensuite repolymérisé en un polymère qui ne présente aucune différence avec un polyester, en particulier un PET, vierge, autorisant ainsi tous les usages du PET vierge.

### DESCRIPTION DÉTAILLÉE DE L'INVENTION

### Charge

Le procédé selon l'invention est alimenté par une charge polyester comprenant au moins un polyester, c'est-à-dire un polymère dont le motif de répétition de la chaine principale contient une fonction ester et comprenant du polyéthylène téréphtalate (PET) opaque. Ladite charge polyester est avantageusement constituée de polyesters recyclés.

Le PET, appelé polyéthylène téréphtalate ou poly(téréphtalate d'éthylène), est un polymère obtenu par la polycondensation de l'acide téréphtalique (PTA) avec l'éthylène glycol de formule chimique : 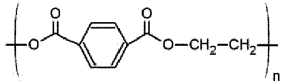 où n représente le nombre de motifs dans le PET. Dans la suite du texte, on entend par moles de diester dans ladite charge polyester le nombre de moles de motif -[0-CO-O-(C₆H₄)-CO-O-CH₂-CH₂]-, qui est le motif diester issu de la réaction du PTA et de l'éthylène glycol, dans le PET compris dans ladite charge polyester.

De préférence, ladite charge polyester comprend au moins un PET choisi parmi le PET opaque, foncé, multicouche et leur mélange. De manière plus préférée, ladite charge polyester comprend au moins 10% en poids de PET opaque, de manière très préférée au moins 15% poids de PET opaque, ledit PET opaque étant avantageusement du PET opaque recyclé.

Ladite charge comprend avantageusement de 0,1% à 10% poids de pigment, avantageusement de 0,1 à 5% poids. Elle comprend avantageusement de 0,05% à 1% de colorants, avantageusement de 0,05 à 0,2% poids.

Ladite charge polyester peut également comprendre jusqu'à 2% poids d'impuretés telles que des métaux, d'autres plastiques (PP, PEHD...), du carton ou papier, du bois ou des minéraux... La charge polyester peut également comprendre des éléments utilisés comme catalyseur de polymérisation et comme agents stabilisants dans les procédés de production de PET, tels que l'antimoine, le titane, l'étain.

Les polyesters, avantageusement recyclés, compris dans ladite charge sont avantageusement lavés et broyés de manière à former une charge polyester constituée de paillettes dont la plus grande longueur maximale est inférieure à 10 cm, préférentiellement comprise entre 5 et 25 mm.

### Étape a) de conditionnement

Ledit procédé selon l'invention comprend une étape a) de conditionnement alimentée par ladite charge polyester.

Ladite étape a) permet de chauffer et de mettre en pression ladite charge polyester aux conditions opératoires de l'étape b) de dépolymérisation.

La charge est progressivement chauffée à une température supérieure à sa température de fusion de manière à devenir liquide. Avantageusement, au moins 80% poids de la charge est sous forme liquide à l'issue de l'étape a), très avantageusement au moins 90% poids, préférentiellement au moins 95% poids à l'issue de l'étape a). La température de ladite étape a) est avantageusement comprise entre 225 et 275°C. Cette température est maintenue la plus faible possible pour minimiser la dégradation thermique du polyester.

Avantageusement, ladite étape a) comprend une section de convoyage à vis, dite section d'extrusion, alimentée par ladite charge polyester.

Le temps de séjour dans ladite section d'extrusion, défini comme le volume de ladite section divisé par le débit volumique de charge est avantageusement inférieur à 15 min, de préférence inférieur à 10 min, et de manière préférée inférieure à 2 min.

Ladite section d'extrusion est avantageusement connectée à un système d'extraction sous vide de manière à éliminer des impuretés telles que des gaz dissous, des composés organiques légers et/ou de l'humidité présents dans la charge. Ladite section d'extrusion peut également avantageusement comprendre un système de filtration pour éliminer des particules solides de taille supérieure à 40 µm, de préférence de taille comprise entre 3 et 40 µm, telles que des particules de sable.

Ladite charge polyester est avantageusement mise en contact avec au moins une fraction de l'effluent diol issu de l'étape c), avantageusement au sein de ladite section d'extrusion. Cette mise en contact a pour effet d'initier la réaction de dépolymérisation avant l'introduction dans l'étape b) de dépolymérisation. Dans ce cas, on parle de section d'extrusion réactive. L'effluent diol issu de l'étape c) peut avantageusement être surchauffé préalablement à son alimentation dans l'étape a) afin de faciliter la mise en température de la charge polyester. Le nombre de moles de diol provenant de l'étape c) par moles de diester dans ladite charge polyester est avantageusement inférieur à 1,0 et de manière préférée inférieur à 0,5.

Ladite charge polyester peut également avantageusement être alimentée en mélange avec une fraction de l'effluent impuretés lourdes issu de l'étape d), ladite fraction ayant préférentiellement été purifiée dans une étape de filtration.

### Étape b) de dépolymérisation

Le procédé selon l'invention comprend une étape de dépolymérisation par glycolyse alimentée au moins par l'effluent de ladite étape a) et par un appoint de diol, opérée à une température comprise entre 200 et 400°C, de préférence entre 230 et 350°C, de manière préférée entre 250°C et 300°C, en phase liquide, avec de 1 à 20 moles de diol par mole de diester dans ladite charge polyester, de préférence de 3 à 15, et de manière préférée de 5 à 10 moles par mole, et un temps de séjour dans ladite étape b) compris entre 0,1 et 5 h de préférence entre 0,5 et 3 h.

La pression d'opération de ladite étape b) est déterminée de manière à maintenir le système réactionnel en phase liquide. Cette pression est d'au moins 0,1 MPa, préférentiellement au moins 0,4 MPa. Par système réactionnel, on entend l'ensemble des constitués et phases présents au sein de ladite étape b) issus de l'alimentation de ladite étape.

Le temps de séjour est défini comme le rapport du volume de liquide de ladite section réactionnelle sur la somme du débit volumique de la charge polyester et de l'appoint de diol.

Le diol est avantageusement du monoéthylène glycol.

Ladite étape b) de dépolymérisation comprend avantageusement une ou plusieurs sections réactionnelles. Chaque section réactionnelle peut être mise en oeuvre dans tout type de réacteur connu de l'homme du métier permettant de réaliser une réaction de dépolymérisation ou de trans-estérification, de préférence, dans un réacteur agité par un système d'agitation mécanique ou/et par boucle de recirculation ou/et par fluidisation. Ledit réacteur peut comprendre un fond conique permettant de purger les impuretés.

La réaction de glycolyse peut être réalisée en présence ou non d'un catalyseur. Lorsque la réaction de glycolyse est réalisée en présence d'un catalyseur, ce dernier peut être homogène ou hétérogène et choisi parmi les catalyseurs d'estérification connus de l'Homme du métier tels que les complexes oxydes et sels d'antimoine, d'étain, de titane, les alkoxydes de métaux des groupes (I) et (IV) de la classification périodique des éléments, les peroxydes organiques, les oxydes métalliques acido-basiques.

Un catalyseur hétérogène préféré comprend avantageusement au moins 50% masse par rapport à la masse totale du catalyseur, préférentiellement au moins 70% masse, avantageusement au moins 80% masse, très avantageusement au moins 90% masse, et façon encore plus avantageuse au moins 95% masse d'une solution solide constituée d'au moins une spinelle de formule ZₓAl₂O₍₃₊ₓ₎ dans laquelle x est compris entre 0 (borne exclue) et 1, et Z est choisi parmi Co, Fe, Mg, Mn, Ti, Zn, et comprenant au plus 50% masse d'alumine et d'oxyde de l'élément Z. Ledit catalyseur hétérogène préféré contient avantageusement au plus 10% masse de dopants choisis parmi le silicium, le phosphore et le bore pris seul ou en mélange. Par exemple, et de manière non limitative, ladite solution solide peut être constituée d'un mélange de spinelle ZnAl₂O₄ et de spinelle CoAl₂O₄, ou bien être constituée d'un mélange de spinelle ZnAl₂O₄, de spinelle MgAl₂O₄ et de spinelle FeAl₂O₄, ou bien être constituée uniquement de spinelle ZnAl₂O₄.

L'arrangement particulier dans lequel ledit catalyseur hétérogène préféré est mis en oeuvre a pour avantage une excellente conversion du PET par glycolyse en BHET. De plus, le catalyseur hétérogène de cet arrangement particulier a pour propriété surprenante de capter les impuretés, en particulier les colorants, les additifs et les substances catalytiques utilisées pour la polymérisation et présentes dans le PET traité dans le procédé selon l'invention, telles que l'antimoine, le magnésium, le manganèse, le zinc, le titane, le phosphore, ce qui simplifie les étapes ultérieures de purification du BHET en vue de sa réutilisation dans un procédé de polymérisation.

De préférence, ladite étape de dépolymérisation est réalisée sans catalyseur.

Ladite étape de dépolymérisation est avantageusement réalisée en présence d'un agent adsorbant solide sous forme de poudre ou mis en forme, dont la fonction est de capter au moins une partie des impuretés colorées, soulageant ainsi l'étape e) de décoloration. Ledit agent adsorbant solide est avantageusement un charbon actif.

La réaction de glycolyse permet de convertir la charge polyester en monomères et oligomères d'esters, avantageusement le PET en monomère Bis(2-Hydroxyethyl) téréphtalate (BHET) et oligomères de BHET. La conversion de la charge polyester dans ladite étape de dépolymérisation est supérieure à 50 %, de préférence supérieure à 70 %, de manière préférée supérieure à 85%. Le rendement molaire en BHET est supérieur à 50 %, de préférence supérieur à 70%, de manière préférée supérieur à 85%. Le rendement molaire en BHET correspond au débit molaire de BHET en sortie de ladite étape b) sur le nombre de moles de diester dans la charge polyester alimentant ladite étape b).

Une boucle interne de recirculation est avantageusement mise en oeuvre dans l'étape b), c'est-à-dire le soutirage d'une fraction du système réactionnel, la filtration de cette fraction, et la réinjection de ladite fraction dans ladite étape b). Cette boucle interne permet d'éliminer les impuretés solides éventuellement comprises dans le liquide réactionnel.

### Étape c) de séparation du diol

Le procédé selon l'invention comprend une étape de séparation du diol alimentée au moins par l'effluent de l'étape b), opérée à une température comprise entre 100 et 250°C, à une pression inférieure à celle de l'étape b) et produisant un effluent diol et un effluent riche en monomères liquide.

L'étape c) a pour fonction principale de récupérer tout ou partie du diol non réagi.

L'étape c) est opérée à une pression inférieure à celle de l'étape b) de manière à vaporiser une fraction de l'effluent de l'étape b) en un effluent gaz et un effluent liquide. Ledit effluent liquide constitue l'effluent riche en monomères liquides. L'effluent gaz, constitué à plus de 50% pds de diol, de préférence plus de 70% pds, de manière préférée plus de 90% pds, constitue un effluent diol gazeux qui est condensé en ledit effluent diol.

L'étape c) est avantageusement mise en oeuvre dans une succession de sections de séparation gaz-liquide, avantageusement de 1 à 5 sections de séparation successives, très avantageusement de 3 à 5 séparations successives. L'effluent liquide de la section antérieure alimente la section ultérieure. L'ensemble des effluents gaz est condensé pour constituer l'effluent diol. L'effluent liquide issu de la dernière section de séparation gaz-liquide constitue l'effluent riche en monomères liquide.

La température et la pression de la section ultérieure sont inférieures à celles de la section antérieure de manière à ce que l'effluent gaz sortant de la section antérieure puisse, en se condensant, rebouillir une partie de l'effluent liquide de la section ultérieure. Dans cette configuration, l'apport de chaleur pour récupérer le diol est minimisé.

L'étape c) est opérée de telle sorte que la température des effluents liquides soit maintenue au-dessus de la valeur en dessous de laquelle le monomère de polyester précipite, et en dessous d'une valeur haute, dépendant du ratio molaire diol/monomère, au-dessus de laquelle le monomère se re-polymérise de manière significative. La température dans l'étape c) est comprise entre 100 et 250°C, de préférence entre 110 et 220°C, de manière préférée entre 120 et 210 °C. L'opération en une succession de séparations gaz-liquide, avantageusement en une succession de 1 à 5, préférentiellement de 3 à 5 séparations successives, est particulièrement avantageuse car elle permet d'ajuster dans chaque séparation la température de l'effluent liquide répondant aux contraintes précitées, ce qui est particulièrement important du fait de la présence de PET opaque dans la charge polyester, les pigments utilisés pour opacifier le PET pouvant avoir une action catalytique dans la réaction de polymérisation du PET.

La pression dans l'étape c) est ajustée pour permettre l'évaporation du diol à une température minimisant la re-polymérisation et permettant une intégration énergétique optimal. Elle est généralement comprise entre 0,00001 et 0,2 MPa, de préférence entre 0,00004 et 0,15 MPa, de manière préférée entre 0,00004 et 0,1 MPa.

La ou les section(s) de séparation sont avantageusement agitée(s) par toute méthode connue de l'homme du métier.

L'effluent diol peut contenir d'autres composés comme des colorants, des alcools légers, de l'eau, du diéthylène glycol. Au moins une fraction de l'effluent diol est avantageusement recyclé vers l'étape a) et/ou l'étape b), avantageusement en mélange avec un appoint en diol externe au procédé selon l'invention.

Tout ou partie dudit effluent diol peut être traité dans une étape de purification préalablement à son recyclage vers les étapes a) et/ou b) et/ou son utilisation en mélange dans l'étape d). Cette étape de purification peut comprendre, de manière non exhaustive, une adsorption sur solide (par exemple sur charbon actif) pour éliminer les colorants et une ou plusieurs distillations pour séparer les impuretés comme le diéthylène glycol, l'eau et d'autres alcools.

### Étape d) de séparation du monomère

Le procédé selon l'invention comprend une étape d) de séparation de l'effluent riche en monomères issu de l'étape c) en un effluent impuretés lourdes et un effluent monomères pré-purifié opérée à une température inférieure à 250°C, de manière préférée inférieure à 230 °C, et de façon très préférée inférieure à 200°C, et une pression inférieure à 0,001 MPa, de préférence inférieure à 0,0005 MPa avec un temps de séjour liquide inférieur à 10 min, de préférence inférieur à 5 min, de manière préférée inférieur à 1 min.

Cette étape de séparation a pour objectif de séparer le monomère, qui est vaporisé, des oligomères et du polyester qui restent liquide et captent donc les impuretés lourdes, notamment les pigments, du polymère non converti, des autres polymères éventuellement présents et des catalyseurs de polymérisation, en minimisant la perte en monomères par re-polymérisation. Quelques oligomères peuvent être entrainés avec le monomère.

L'élimination totale des pigments par filtration est particulièrement difficile en raison de la taille très faible desdits pigments. L'effluent riche en monomères issu de l'étape c) comprend avantageusement une teneur total en cations et anions de plus de 50 ppm, très avantageusement de plus de 100 ppm.

Du fait de la présence possible dans la charge polyester de catalyseurs de polymérisation, en particulier si cette charge comprend du PET opaque, cette séparation doit être réalisée avec des temps de séjour liquide très courts et à une température n'excédant pas 250°C. Une séparation par distillation atmosphérique simple n'est donc pas envisageable. Certains pigments utilisés pour opacifier le PET, tels que le TiO₂, sont connus pour catalyser la réaction de polymérisation.

L'étape d) de séparation est avantageusement mise en oeuvre dans un système d'évaporation à film tombant ou film raclé ou par distillation à court trajet à film tombant ou à film raclé. La très faible pression opératoire est nécessaire pour pouvoir opérer l'étape d) à une température inférieure à 250°C, de préférence inférieure à 230°C tout en permettant la vaporisation du monomère.

Un inhibiteur de polymérisation est avantageusement mélangé à l'effluent riche en monomères liquide avant d'être alimenté dans ladite étape d).

Un fluxant est avantageusement mélangé à l'effluent riche en monomères liquide avant d'être alimenté dans ladite étape d) de manière à faciliter l'élimination des impuretés lourdes, notamment des pigments, en fond du système d'évaporation ou de distillation court trajet. Ce fluxant doit avoir une température d'ébullition très supérieur au BHET dans les conditions d'opération de l'étape d). Il peut s'agir par exemple de polyéthylène glycol, ou d'oligomères du PET.

Ledit effluent impuretés lourdes comprend en particulier les pigments, des oligomères et du BHET non séparé. Une fraction dudit effluent impuretés lourdes peut avantageusement être recyclé vers l'étape a) de conditionnement et d'alimentation et/ou vers l'étape b) de dépolymérisation.

Ledit effluent impuretés lourdes subit avantageusement au moins une étape de purification, de manière préférée une étape de filtration préalablement à son recyclage de manière à réduire la quantité de pigments et/ou autres impuretés solide. Tout ou partie dudit effluent impuretés lourdes peut également avantageusement être purgé du procédé et envoyé vers un système d'incinération.

Une fraction de l'effluent diol peut avantageusement être mélangée avec l'effluent impuretés lourdes issu de l'étape d) de manière à diminuer la viscosité dudit effluent impuretés lourdes et faciliter son transport vers l'étape a) et/ou l'étape b), et éventuellement son traitement dans une étape optionnelle de filtration.

Ledit effluent monomère pré-purifié est avantageusement envoyé dans une section de séparation gaz-liquide, opérée dans tout équipement connu de l'Homme du Métier, à une température comprise entre 100 et 250°C, de préférence entre 110 et 200°C, et de manière préférée entre 120 et 180°C, et à une pression comprise entre 0,00001 et 0,1 MPa, de préférence entre 0,00001 et 0,01 MPa, et de manière préférée entre 0,00001 et 0,001 MPa. Ladite section de séparation permet de séparer un effluent diol gazeux et un effluent monomère pré-purifié liquide. Ladite séparation gaz-liquide permet de réduire encore la quantité de diol restant dans l'effluent monomère pré-purifié en récupérant dans ledit effluent diol gazeux plus de 50 % pds, de préférence plus 70 % pds, de manière préférée plus de 90% pds du diol entrainé dans l'étape d) avec l'effluent monomère pré-purifié. La quantité de monomère entrainé dans ledit effluent diol gazeux est de préférence inférieure à 1 % pds, de manière préférée inférieure à 0,1 % pds et de manière plus préférée inférieure à 0.01% pds de la quantité de monomère présent dans l'effluent monomère pré-purifié. Ledit effluent diol gazeux est ensuite avantageusement condensé, éventuellement prétraité dans une étape de purification et recyclé avec l'effluent diol issu de l'étape c) vers l'étape a) et/ou l'étape b) et/ou en mélange dans l'étape d).

### Étape e) de décoloration

Le procédé selon l'invention comprend une étape de décoloration de l'effluent monomères pré-purifié, opérée à une température comprise entre 100 et 250°C, de préférence entre 110 et 200°C, et de manière préférée entre 120 et 180°C, et à une pression comprise entre 0,1 et 1,0 MPa, de préférence entre 0,2 et 0,8 MPa, et de manière préférée entre 0,3 et 0,5 MPa en présence d'un adsorbant et produisant un effluent monomères purifié.

Ledit adsorbant peut être tout adsorbant connu de l'Homme du métier apte à capter les colorants, telles que du charbon actif, des argiles, avantageusement un charbon actif.

L'effluent monomères pré-purifié est avantageusement mélangé avec une fraction de l'effluent diol issu de l'étape c) ou avec un appoint en diol externe au procédé selon l'invention.

L'effluent monomère purifié alimente avantageusement une étape de polymérisation connue de l'Homme du métier en vue de produire du PET que rien ne distingue du PET vierge, avantageusement en aval de l'alimentation en éthylène glycol, en acide téréphtalique ou en diméthyltéréphtalate suivant l'étape de polymérisation retenue. L'alimentation de l'effluent monomère purifié dans une étape de polymérisation permet de diminuer d'un débit équivalent l'alimentation en diméthyltéréphtalate ou en acide téréphtalique.

### EXEMPLES

### EXEMPLE 1 - CONFORME

*Cet exemple illustre l'utilisation du procédé selon l'invention avec une charge comprenant 20% poids de PET opaque.*

4 kg/h de paillettes issues d'une charge PET recyclée, broyée et lavée, constituée à 20% poids de PET opaque et comprenant 5% poids de pigment TiO₂, et 12.9 kg/h d'éthylène glycol (MEG), sont portés à une température de 250°C puis injectés dans un réacteur agité maintenu à une pression de 0,4 MPa. Le temps de séjour, défini comme le rapport du volume liquide du réacteur par la somme des débits volumiques liquides entrant dans le réacteur, est fixé à 5 h. A la sortie du réacteur, l'effluent réactionnel est constitué de 69,06 % pds de MEG, 27,74 % pds de BHET, 2,96 % pds de dimère de BHET, et 0,24% pds de TiO₂.

L'éthylène glycol présent dans l'effluent réactionnel est séparé par évaporation dans une succession de 4 ballons à des températures allant de 210°C à 130°C et des pressions de 0,12 MPa à 0,001 MPa. A l'issue de cette étape d'évaporation, un flux de MEG de 11,1 kg/h et un flux liquide riche en BHET de 5,84 kg/h sont récupérés. Le flux de MEG est constitué quasi exclusivement d'éthylène glycol et peut ainsi être recyclé vers le réacteur de dépolymérisation. Le flux liquide riche en BHET est constitué de 80,50% pds de BHET, 8,52 % pds de dimère de BHET, 10,3 % pds de MEG et 0,68% pds de TiO₂.

Le flux liquide riche en BHET est ensuite injecté dans un évaporateur à film raclé à une température de 220°C et une pression de 50 Pa. Le temps de séjour dans l'évaporateur à film raclé est de 1 min. Un effluent gaz avec un débit de 5,2 kg/h est récupéré en tête de l'évaporateur à film raclé. Il est constitué de 88,5 % pds de BHET et 11,5 % pds de MEG et est exempt de trace de TiO₂. Un résidu lourd avec un débit de 0,64 kg/h est récupéré en fond de l'évaporateur à film raclé et est constitué de 93,75% pds d'oligomères de BHET et 6,25 % pds de TiO₂.

L'effluent gaz est condensé à 130°C pour donner un flux liquide de BHET pré-purifié. Le flux liquide de BHET pré-purifié est comprimé jusqu'à 0,5 MPa et alimente ensuite un lit fixe de charbon actif ayant une capacité d'adsorption égale à 5% de sa masse. A l'issue de cette étape, on obtient un flux liquide de BHET décoloré et dépigmenté, qui est réinjecté dans une étape de polymérisation connue de l'homme du métier en vue de produire du PET vierge.

### EXEMPLE 2 - CONFORME

*Cet exemple illustre l'utilisation du procédé selon l'invention avec une charge 100% PET opaque.*

4 kg/h de paillettes issues d'une charge PET recyclée, broyée et lavée, constituée à 100% de PET opaque dont 5%pds de pigment TiO₂, et 12,9 kg/h d'éthylène glycol (MEG), sont portés à une température de 250°C puis injectés dans un réacteur agité maintenu à une pression de 0,4 MPa. Le temps de séjour, défini comme le rapport du volume liquide du réacteur par la somme des débits volumiques liquides entrant dans le réacteur, est fixé à 5 h. A la sortie du réacteur, l'effluent réactionnel est constitué de 69,82 % pds de MEG, 26,63 % pds de BHET, 2,37 % pds de dimère de BHET, et 1,18 % pds de TiO₂.

L'éthylène glycol présent dans l'effluent réactionnel est séparé par évaporation dans une succession de 4 ballons à des températures allant de 210°C à 130°C et des pressions de 0,12 MPa à 0,001 MPa. A l'issue de cette étape d'évaporation, un flux de MEG de 11,2 kg/h et un flux liquide riche en BHET de 5,7 kg/h sont récupérés. Le flux de MEG est constitué quasi exclusivement d'éthylène glycol et peut ainsi être recyclé vers le réacteur de dépolymérisation. Le flux liquide riche en BHET est constitué de 78,9% pds de BHET, 7,0 % pds de dimère de BHET, 10,5 % pds de MEG et 3,51 % pds de TiO₂.

Le flux liquide riche en BHET est ensuite injecté dans un évaporateur à film raclé à une température de 220°C et une pression de 50 Pa. Le temps de séjour dans l'évaporateur à film raclé est de 1 min. Un effluent gaz avec un débit de 5,2 kg/h est récupéré en tête de l'évaporateur à film raclé. Il est constitué de 88 % pds de BHET et 12 % pds de MEG et est exempt de trace de TiO₂. Un résidu lourd avec un débit de 0,64 kg/h est récupéré en fond de l'évaporateur à film raclé et est constitué de 75% pds d'oligomères de BHET et 25 % pds de TiO₂.

L'effluent gaz est condensé à 130°C pour donner un flux liquide de BHET pré-purifié. Le flux liquide de BHET pré-purifié est comprimé jusqu'à 0,5 MPa et alimente ensuite un lit fixe de charbon actif ayant une capacité d'adsorption égale à 5% de sa masse. A l'issue de cette étape, on obtient un flux liquide de BHET décoloré et dépigmenté, qui est réinjecté dans une étape de polymérisation connue de l'homme du métier en vue de produire du PET vierge.

### EXEMPLE 3 - NON CONFORME

*Cet exemple illustre l'utilisation d'un procédé selon l'art antérieur (*JP3715812*) avec une charge comprenant du PET opaque.*

4 kg/h de paillettes issues d'une charge PET recyclée, broyée et lavée, constituée à 20% poids de PET opaque et comprenant 5% poids de pigment TiO₂, et 12.9 kg/h d'éthylène glycol (MEG), sont portés à une température de 250°C puis injectés dans un réacteur agité maintenu à une pression de 0,4 MPa. Le temps de séjour, défini comme le rapport du volume liquide du réacteur par la somme des débits volumiques liquides entrant dans le réacteur, est fixé à 5 h. A la sortie du réacteur, l'effluent réactionnel est constitué de 69,06 % pds de MEG, 27,74 % pds de BHET, 2,96 % pds de dimère de BHET, et 0,24% pds de TiO₂.

Le document JP3715812 enseigne qu'il est nécessaire et important de procéder à une pré-purification, c'est-à-dire une étape de filtration (40-100 microns) suivie d'une étape de déionisation, avant l'utilisation d'un séparateur à court temps de contact, cette pré-purification permettant d'extraire du flux les espèces favorisant les réactions de repolymérisation et de coloration du BHET.

L'effluent de dépolymérisation est pompé et filtré à 100°C et 0,4 MPa sur un filtre cartouche de 44 microns de porosité (325 mesh), puis refroidi à 50°C et envoyé dans un lit fixe contenant une résine échangeuse d'ions. La pression est suivie en continu en amont du filtre et en aval du lit de résine. La pression augmente lentement dans les premières heures d'opération et la différence de pression entre l'amont du filtre et l'aval du lit de résine reste inférieur à 2 bar, ce qui permet de conserver l'intégrité du lit de résine. Au bout de 12 h d'opération, la pression augmente fortement jusqu'à 8 bar et l'unité est arrêté 30 min après pour cause de bouchage et d'une perte de structure du lit de résine. La différence de pression entre l'amont du filtre et l'aval du lit de résine est mesuré à 6 bar avant le bouchage.

Les enchaînements d'étapes de l'art antérieur mettant en oeuvre une pré-purification par filtration et adsorption sur résine ne permettent donc pas de traiter avec une charge contenant du PET opaque en quantité supérieure à 10%pds.

## Revendications

1. Procédé de dépolymérisation d'une charge polyester comprenant du PET opaque, ladite charge comprenant de 0,1 à 10% poids de pigment, ledit procédé comprenant au moins les étapes suivantes :
a) une étape de conditionnement alimentée par ladite charge polyester ;
b) une étape de dépolymérisation par glycolyse alimentée au moins par l'effluent de l'étape a) et par un appoint de diol, opérée à une température comprise entre 200 et 400°C, avec de 1 à 20 moles de diol par mole de diester dans ladite charge polyester et un temps de séjour du polyester compris entre 0,1 et 5 h convertissant le PET en monomère BHET et oligomères du BHET ;
c) une étape de séparation du diol alimentée au moins par l'effluent de l'étape b), opérée à une température comprise entre 100 et 250°C, à une pression inférieure à celle de l'étape b) et produisant un effluent diol et un effluent riche en monomères liquide, mise en oeuvre dans 1 à 5 sections de séparation gaz-liquide successives, l'effluent liquide de la section antérieure alimentant la section ultérieure, l'ensemble des effluents gaz étant condensé pour constituer l'effluent diol, l'effluent liquide issu de la dernière section de séparation gaz-liquide constituant l'effluent riche en monomères liquide ;
d) une étape de séparation de l'effluent riche en monomères liquides issu de l'étape c) en un effluent impuretés lourdes et un effluent monomères pré-purifié opérée à une température inférieure à 250°C et une pression inférieure à 0,001 MPa avec un temps de séjour liquide inférieur à 10 min et
e) une étape de décoloration de l'effluent monomères pré-purifié, opérée à une température comprise entre 100 et 250°C, et à une pression comprise entre 0,1 et 1,0 MPa en présence d'un adsorbant et produisant un effluent monomères purifié.

2. Procédé selon la revendication 1 dans lequel ladite charge polyester comprend au moins 10% en poids de PET opaque.

3. Procédé selon l'une des revendications précédentes dans laquelle l'étape a) est opérée à une température comprise entre 225 et 275°C.

4. Procédé selon l'une des revendications précédentes dans lequel ladite étape a) comprend une section d'extrusion.

5. Procédé selon l'une des revendications précédentes dans lequel ladite charge polyester est mise en contact avec au moins une fraction de l'effluent diol issu de l'étape c) dans ladite étape a).

6. Procédé selon l'une des revendications précédentes dans lequel ladite étape b) est réalisée en présence d'un adsorbant solide.

7. Procédé selon l'une des revendications précédentes dans lequel ladite étape b) est opérée en présence d'un catalyseur hétérogène comprenant au moins 50% masse par rapport à la masse totale du catalyseur d'une solution solide constituée d'au moins une spinelle de formule ZₓAl₂O₍₃₊ₓ₎ dans laquelle x est compris entre 0 (borne exclue) et 1, et Z est choisi parmi Co, Fe, Mg, Mn, Ti, Zn, et comprenant au plus 50% masse d'alumine et d'oxyde de l'élément Z.

8. Procédé selon l'une des revendications précédentes dans lequel une fraction de l'effluent diol issu de l'étape c) est recyclé vers l'étape b).

9. Procédé selon l'une des revendications précédentes dans lequel ladite étape d) est opérée à une pression inférieure à 0,0005 MPa.

10. Procédé selon l'une des revendications précédentes dans lequel ladite étape d) est opérée avec un temps de séjour liquide inférieur à 1 min.

11. Procédé selon l'une des revendications précédentes dans lequel une fraction dudit effluent impuretés lourdes est recyclé vers l'étape a) de conditionnement et d'alimentation et/ou vers l'étape b) de dépolymérisation.

12. Procédé selon la revendication 11 dans lequel une fraction de l'effluent diol issu de l'étape c) est mélangée avec l'effluent impuretés lourdes issu de l'étape d).

13. Procédé selon l'une des revendications précédentes dans lequel ledit effluent monomère pré-purifié issu de l'étape d) est envoyé dans une section de séparation gaz-liquide, opérée à une température comprise entre 100 et 250°C, et à une pression comprise entre 0,00001 et 0,1 MPa.

14. Procédé selon l'une des revendications précédentes dans lequel l'effluent monomère purifié alimente une étape de polymérisation en vue de produire du PET.

## Patentansprüche

1. Verfahren zur Depolymerisation einer opakes PET umfassenden Polyester-Beschickung, wobei die Beschickung 0,1 bis 10 Gew.-% Pigment umfasst, wobei das Verfahren mindestens die folgenden Schritte umfasst:
a) einen Aufbereitungsschritt, der mit der Polyester-Beschickung beschickt wird;
b) einen Schritt der Depolymerisation durch Glykolyse, der mindestens mit dem Abstrom von Schritt a) und durch eine Zugabe von Diol beschickt wird und bei einer Temperatur zwischen 200 und 400 °C mit 1 bis 20 Mol Diol pro Mol Diester in der Polyester-Beschickung und einer Verweildauer des Polyesters zwischen 0,1 und 5 Std. durchgeführt wird, wodurch das PET in BHET-Monomer und BHET-Oligomere umgewandelt wird;
c) einen Schritt der Abtrennung des Diols, der mindestens mit dem Abstrom von Schritt b) beschickt wird und bei einer Temperatur zwischen 100 und 250 °C, bei einem niedrigeren Druck als derjenige von Schritt b) durchgeführt wird und einen Diolabstrom sowie einen an Monomeren reichen flüssigen Abstrom produziert und der in 1 bis 5 aufeinanderfolgenden Gas/Flüssigkeits-Trennabschnitten durchgeführt wird, wobei der flüssige Abstrom des vorhergehenden Abschnitts den nachfolgenden Abschnitt beschickt, wobei die gesamten Gasabströme kondensiert werden und den Diolabstrom bilden, wobei der aus dem letzten Gas/Flüssigkeits-Trennabschnitt stammende flüssige Abstrom den flüssigen, an Monomeren reichen Abstrom bildet;
d) einen Schritt der Trennung des aus Schritt c) stammenden flüssigen, an Monomeren reichen Abstroms in einen Abstrom schwerer Verunreinigungen und einen Abstrom vorgereinigter Monomere, der bei einer Temperatur von weniger als 250 °C und einem Druck von weniger als 0,001 MPa mit einer Flüssigkeitsverweildauer von weniger als 10 min durchgeführt wird, und
e) einem Schritt der Entfärbung des Abstroms vorgereinigter Monomere, der bei einer Temperatur zwischen 100 und 250 °C und bei einem Druck zwischen 0,1 und 1,0 MPa in Gegenwart eines Adsorptionsmittels durchgeführt wird und einen Abstrom gereinigter Monomere produziert.

2. Verfahren nach Anspruch 1, wobei die Polyester-Beschickung mindestens 10 Gew.-% opakes PET umfasst.

3. Verfahren nach einem der vorhergehenden Ansprüche, wobei Schritt a) bei einer Temperatur zwischen 225 und 275 °C durchgeführt wird.

4. Verfahren nach einem der vorhergehenden Ansprüche, wobei der Schritt a) einen Extrusionsabschnitt umfasst.

5. Verfahren nach einem der vorhergehenden Ansprüche, wobei die Polyester-Beschickung im Schritt a) mit mindestens einer Fraktion des aus Schritt c) hervorgegangenen Diolabstroms in Kontakt gebracht wird.

6. Verfahren nach einem der vorhergehenden Ansprüche, wobei der Schritt b) in Gegenwart eines festen Adsorptionsmittels durchgeführt wird.

7. Verfahren nach einem der vorhergehenden Ansprüche, wobei der Schritt b) in Gegenwart eines heterogenen Katalysators durchgeführt wird, der mindestens 50 Massen-%, bezogen auf die Gesamtmasse des Katalysators, einer festen Lösung umfasst, die aus mindestens einem Spinell der Formel ZₓAl₂O₍₃₊ₓ₎ besteht, wobei x zwischen 0 (Grenzwert ausgeschlossen) und 1 liegt und Z aus Co, Fe, Mg, Mn, Ti, Zn ausgewählt ist, der höchstens 50 Massen-% Aluminiumoxid und Oxid des Elements Z umfasst.

8. Verfahren nach einem der vorhergehenden Ansprüche, wobei eine Fraktion des aus Schritt c) stammenden Diolabstroms zum Schritt b) rückgeführt wird.

9. Verfahren nach einem der vorhergehenden Ansprüche, wobei der Schritt d) bei einem Druck von weniger als 0,0005 MPa durchgeführt wird.

10. Verfahren nach einem der vorhergehenden Ansprüche, wobei der Schritt d) mit einer Flüssigkeitsverweildauer von weniger als 1 min durchgeführt wird

11. Verfahren nach einem der vorhergehenden Ansprüche, wobei eine Fraktion des Abstroms schwerer Verunreinigungen zum Aufbereitungs- und Beschickungsschritt a) und/oder zum Depolymerisationsschritt b) rückgeführt wird.

12. Verfahren nach Anspruch 11, wobei eine Fraktion des aus Schritt c) stammenden Diolabstroms mit dem aus Schritt d) stammenden Abstrom schwerer Verunreinigungen gemischt wird.

13. Verfahren nach einem der vorhergehenden Ansprüche, wobei der aus Schritt d) stammende Abstrom von vorgereinigtem Monomer in einen Gas/Flüssigkeits-Trennabschnitt geleitet wird, der bei einer Temperatur zwischen 100 und 250 °C und bei einem Druck zwischen 0,00001 und 0,1 MPa betrieben wird.

14. Verfahren nach einem der vorhergehenden Ansprüche, wobei mit dem Abstrom von gereinigtem Monomer ein Polymerisationsschritt für die Produktion von PET beschickt wird.

## Claims

1. Process for the depolymerization of a polyester feedstock comprising opaque PET, said feedstock comprising from 0.1% to 10% by weight of pigment, said process comprising at least the following steps:
a) a step of conditioning fed by said polyester feedstock;
b) a step of depolymerization by glycolysis fed at least by the stream from step a) and by an addition of diol, operated at a temperature of between 200 and 400°C, with from 1 to 20 mol of diol per mole of diester in said polyester feedstock and a residence time of the polyester of between 0.1 and 5 h, converting the PET into BHET monomer and BHET oligomers;
c) a step of separation of the diol fed at least by the stream from step b), operated at a temperature of between 100 and 250°C, at a lower pressure than that of step b) and producing a diol stream and a liquid stream rich in monomers, carried out in 1 to 5 successive gas/liquid separation stages, the liquid stream from the preceding stage feeding the next stage, all of the gas streams being condensed to form the diol stream, the liquid stream resulting from the final gas/liquid separation stage constituting the liquid stream rich in monomers;
d) a step of separation of the liquid stream rich in monomers resulting from step c) into a heavy impurities stream and a prepurified monomers stream operated at a temperature of less than 250°C and a pressure of less than 0.001 MPa with a liquid residence time of less than 10 min; and
e) a step of decoloration of the prepurified monomers stream, operated at a temperature of between 100 and 250°C and at a pressure of between 0.1 and 1.0 MPa in the presence of an adsorbent and producing a purified monomers stream.

2. Process according to Claim 1, wherein said polyester feedstock comprises at least 10% by weight of opaque PET.

3. Process according to either of the preceding claims, wherein step a) is operated at a temperature of between 225 and 275°C.

4. Process according to one of the preceding claims, wherein said step a) comprises an extrusion stage.

5. Process according to one of the preceding claims, wherein said polyester feedstock is brought into contact with at least a fraction of the diol stream resulting from step c) in said step a).

6. Process according to one of the preceding claims, wherein said step b) is executed in the presence of a solid adsorbent.

7. Process according to one of the preceding claims, wherein said step b) is operated in the presence of a heterogeneous catalyst comprising at least 50% by weight, relative to the total weight of the catalyst, of a solid solution consisting of at least one spinel of formula ZₓAl₂O₍₃₊ₓ₎ wherein x is between 0 (not inclusive) and 1, and Z is chosen from Co, Fe, Mg, Mn, Ti or Zn, and comprising at most 50% by weight of alumina and of oxide of the element Z.

8. Process according to one of the preceding claims, wherein a fraction of the diol stream resulting from step c) is recycled to step b).

9. Process according to one of the preceding claims, wherein said step d) is operated at a pressure of less than 0.0005 MPa.

10. Process according to one of the preceding claims, wherein said step d) is operated with a liquid residence time of less than 1 min.

11. Process according to one of the preceding claims, wherein a fraction of said heavy impurities stream is recycled to the conditioning and feeding step a) and/or to the depolymerization step b).

12. Process according to Claim 11, wherein a fraction of the diol stream resulting from step c) is mixed with the heavy impurities stream resulting from step d) .

13. Process according to one of the preceding claims, wherein said prepurified monomer stream resulting from step d) is sent to a gas/liquid separation stage, this separation being operated at a temperature of between 100 and 250°C and at a pressure of between 0.00001 and 0.1 MPa.

14. Process according to one of the preceding claims, wherein the purified monomer stream feeds a polymerization step for the purpose of producing PET.
